# EUROPEAN PATENT APPLICATION

(11) **EP 4 446 108 A1**
(43) Date of publication of application: **16.10.2024**
(21) Application number: 22915696.3
(22) Date of filing: 12.12.2022
(51) Int. Cl.: B32B 7/05, A61F 13/49, A61F 13/51, B32B 27/12

(54) **LAMINATE**

(30) Priority: 27.12.2021 JP 2021212033
(71) Applicant: Zuiko Corporation, Ibaraki-shi Osaka 5670082 (JP)
(72) Inventor: KOSHIJIMA Miwa, Ibaraki-shi, Osaka 567-0082 (JP)
(74) Representative: Hasegawa, Kan
(86) International application number: PCT/JP2022/045638
(87) International publication number: WO 2023/127472

(57) **Abstract**

A laminate in which a thermoplastic elastomer film having greater stretchability in a stretch direction than in a width direction orthogonal to the stretch direction and nonwoven fabric sheets are laminated to each other, wherein the elastomer film and the sheets are laminated by being heat fused to each other at bonded portions intermittently arranged in the width direction and the stretch direction, and the sum of lengths of the bonded portions in the width direction in at least one side edge portion in the width direction per unit area of the laminate in that side edge portion is greater than the sum of lengths of the bonded portions in the width direction in a non-side edge portion in the width direction per unit area of the laminate in that non-side edge portion.

## Description

### TECHNICAL FIELD

The present invention relates to a stretchable laminate that can be utilized for parts of disposable wearable articles such as disposable diapers and disposable pants.

### BACKGROUND ART

A stretchable composite sheet, that is, a stretchable laminate, is formed by extruding into a film and cooling an elastic resin material having a thermoplastic resin as its main component, then laminating a base sheet on at least one side of the film, and intermittently heat fusing the film and the base sheet.

### CITATION LIST

### Patent Literature

Patent Document 1: WO 2020/090733

### SUMMARY OF INVENTION

When forming such a stretchable composite sheet, the resin material, which is in a molten state just after the film is extruded, shrinks in its width direction perpendicular to the extrusion direction due to necking, and after cooling, the thickness of the film at both side edge portions in the width direction ends up becoming thick, with the result that the elongation stress (a mechanical property) of the stretchable composite sheet becomes different in the width direction.

In order to obtain a stretchable composite sheet with uniform elongation stress, it may be conceivable to cut off all the parts of the composite sheet in the regions where the thickness of the film is thick. However, such a process increases material waste.

It is an object of the present invention to obtain a stretchable composite sheet, that is, a stretchable laminate, with which material waste can be reduced and which has smaller variations in elongation stress.

A laminate of the present invention is a laminate W comprising a thermoplastic elastomer film F having greater stretchability in a stretch direction Ds than in a width direction Dw orthogonal to the stretch direction Ds and nonwoven fabric sheet S1, S2 that are laminated to each other,
wherein the elastomer film F and the sheet S1, S2 are laminated by being heat fused to each other at bonded portions 8 intermittently arranged in the width direction Dw and the stretch direction Ds, and
the sum of lengths of each of the bonded portions 8 in the width direction Dw in at least one side edge portion A_{E} in the width direction Dw per unit area of the laminate in that side edge portion A_{E} is greater than the sum of lengths of each of the bonded portions 8 in the width direction Dw in a non-side edge portion Ac in the width direction Dw per unit area of the laminate W in that non-side edge portion Ac.

The pair of nonwoven fabric sheets and the elastomer film F are heat fused to each other at the bonded portions 8. The elastomer film F around the bonded portions 8 breaks during the heat fusion or breaks during re-elongation such as during wearing, so that holes appear in an elongated state of the elastomer film F. The holes greatly extend in the stretch direction Ds with a small tensile force because the elastomer film is missing in them.

The magnitude of this extension becomes greater the longer the length of the holes in the width direction Dw is. The lengths of the holes in the width direction Dw approximate the lengths of the bonded portions 8. Consequently, the greater the sum of the lengths of the bonded portions 8 in the width direction Dw is, the greater the laminate stretches in the stretch direction Ds with a small tensile force.

Furthermore, since it is also not necessary to cut off all the portions of the laminate in the regions where the thickness of the film is thick, there is also no significant increase in material waste.

It will be noted that from the standpoint of improving the appearance of the product for example, part of the side edge portion of the laminate may be cut off.

In the present invention, "side edge portion" means a region that is near an edge of the laminate and in which the sum of the lengths of the bonded portions in the width direction Dw is greater than the sum of the lengths of other bonded portions in the width direction, and "non-side edge portion" means a portion other than the side edge portion.

### BRIEF DESCRIPTION OF DRAWINGS

FIG. 1 is a perspective view showing an embodiment of the laminate of the present invention. It will be noted that in FIG. 1, for convenience of drawing, a nonwoven fabric sheet on the upper side is depicted flat. Furthermore, in FIG. 1 and other figures, an elastomer film and sheets are enlarged in the thickness direction and depicted thicker than they really are to allow the structure of the laminate to be more easily understood.
FIG. 2A is a cross-sectional view of the laminate, FIG. 2B is a partial plan view of the laminate, and FIG. 2C is an enlarged partial cross-sectional view of a bonded portion and its surroundings.
FIG. 3 is a conceptual diagram showing an example of a method of manufacturing a wearable article.
FIG. 4A is a plan view showing an example of the wearable article, FIG. 4B is an enlarged plan view showing part of a waist portion, and FIG. 4C is a longitudinal cross-sectional view of the same. It will be noted that in FIG. 1 to FIG. 4C, the bonded portions are shaded in gray and the elastomer film is given a dot pattern to allow the drawings to be more easily seen.
FIG. 5 is an enlarged plan view showing part of the waist portion.
FIG. 6A is an enlarged plan view showing first bonded portions, FIG. 6B is a cross-sectional view of the same, FIG. 6C is an enlarged plan view showing third bonded portions, and FIG. 6D is a cross-sectional view of the same.
FIG. 7A and FIG. 7B are layout diagrams showing an embodiment of a process of forming a film pass line.
FIG. 8A and FIG. 8B are layout diagrams showing an embodiment of a process of forming a film pass line.
FIG. 9A and FIG. 9B are schematic layout diagrams showing an embodiment of a method and apparatus for manufacturing the laminate of the present invention.

### DESCRIPTION OF EMBODIMENTS

Preferably, a length L of each of the bonded portions 8 in the width direction Dw is longer than that of the bonded portions 8 in the stretch direction Ds.

The length of the bonded portions in the stretch direction may be made longer than their length in the width direction, but in this case, the effect of reducing variations in elongation stress in the width direction is reduced. In contrast, by making the length of the bonded portions in the width direction longer than their length in the stretch direction, the effect of reducing variations in elongation stress in the width direction is easy to obtain.

Preferably, lengths L₁, L₂ in the width direction Dw in the side edge portion A_{E} are set longer than a length L₃ in the width direction Dw in the non-side edge portion Ac.

In this case, the lengths in the width direction in the side edge portion become longer, and it becomes easy to further reduce variations in the elongation stress in the width direction.

The degree of necking is greater closer to the edge of the laminate, and the elastomer film is often thicker closer to the edge.

For that reason, for example, at least two rows of first bonded portions 8₁ and second bonded portions 8₂ intermittently arranged in the stretch direction Ds may be provided in the side edge portion A_{E},
the first bonded portions 8₁ may be provided in positions closest to an edge E of the side edge portion A_{E}, and
the row of the second bonded portions 8₂ may be provided next to the row of the first bonded portions 8₁.

In this case, preferably a length L₁ of the first bonded portions 8₁ in the width direction Dw is longer than a length L₂ of the second bonded portions 8₂ in the width direction Dw.

In this way, because the first bonded portions that are in positions closest to the edge of the laminate have a longer length in the width direction compared with the second bonded portions in the adjacent row, variations in the elongation stress of the thick elastomer film can be further reduced closer to the edge.

Even more preferably, a plurality of third bonded portions 8₃ are provided in the non-side edge portion Ac, and the length L₂ of the second bonded portions 8₂ in the width direction Dw is longer than a length L₃ of the third bonded portions 8₃ in the width direction Dw.

In this case, variations in elongation stress between the side edge portion and the non-side edge portion can be further reduced.

Preferably, arrangement pitch P₁, P₂ of the bonded portions 8 in the stretch direction Ds in the side edge portion A_{E} is set smaller than an arrangement pitch P₃ of the bonded portions 8 in the stretch direction Ds in the non-side edge portion Ac.

By setting the pitches of the bonded portions in this way, the sum of said lengths in the side edge portions can be set greater than the sum of said lengths in the non-side edge portion.

Preferably, an arrangement pitch P₁ of the first bonded portions 8₁ in the stretch direction Ds is smaller than an arrangement pitch P₂ of the second bonded portions 8₂ in the stretch direction Ds.

In this way, because the first bonded portions that are in positions closest to the edge of the laminate have a small arrangement pitch compared with the second bonded portions in the row next to them, variations in the elongation stress of the thick elastomer film can be reduced closer to the edge.

Preferably, an arrangement pitch P₂ of the second bonded portions 8₂ in the stretch direction Ds is smaller than an arrangement pitch P₃ of the third bonded portions 8₃ in the stretch direction Ds in one row in the non-side edge portion Ac.

In this case, variations in elongation stress between the side edge portion and the non-side edge portion can be further reduced.

Preferably, a first area ratio α1 of the area of the bonded portions 8 in at least one side edge portion A_{E} in the width direction Dw to the unit area of the laminate in that side edge portion A_{E} is greater than a second area ratio α2 of the area of the bonded portions 8 in a non-side edge portion Ac in the width direction Dw to the unit area of the laminate W in that non-side edge portion Ac.

Here, the bonded portions in the present invention will be described.

At each of the bonded portions, the elastomer film and the sheets are heat fused to each other, and because of this, a state in which the film and the sheets are laminated is maintained. The bonded portions at which the two are heat fused to each other are in a relaxed state in which pre-stress that occurred during manufacture is removed. Consequently, the bonded portions mitigate the ease with which the laminate shrinks.

Furthermore, at the bonded portions, some of the resin component of the elastomer film penetrates between the fibers of the nonwoven fabric sheets, and the mechanically effective thickness of the film becomes smaller. For that reason, resistance to stretching is small at the bonded portions compared with their surrounding portions. Consequently, it is easy for the laminate to stretch when it is pulled in the stretch direction.

Since the bonded portions have the property of mitigating the ease with which the laminate shrinks and making it easier for the laminate to stretch as described above, in a no-load state, the bonded portions can be said to be in a "relaxed state."

Here, the first area ratio α1 of the bonded portions in the side edge portion is greater than the second area ratio α2 in the non-side edge portion. In other words, the area ratio of the bonded portions is large in the side edge portion compared with in the non-side edge portion. Because the area ratio of the bonded portions is large in this way, even if the side edge portion is thick due to necking compared with the non-side edge portion, the bonded portions in the "relaxed state" can lower the resistance of the side edge portion and bring it closer to the resistance of the non-side edge portion. Consequently, variations in elongation stress become smaller in the width direction of the laminate.

Preferably, each bonded portion 8 of a plurality of bonded portions 8 at which the nonwoven fabric sheets S 1, S2 and the elastomer film F are heat fused to each other includes a fused region α at which the sheets S 1, S2 and the elastomer film F are heat fused to each other, and
the elastomer film F is in a broken state at boundary lines 80 defining the fused regions α.

In this case, as mentioned above, holes 8H defined by boundary lines 80 spread wide when the laminate stretches in the stretch direction Ds. For that reason, the side edge portion having the bonded portions 8 that are long in the width direction Dw easily stretches in spite of the elastomer film F being thick.

In the laminate, a thickness Th1 of the elastomer film F in the at least one side edge portion A_{E} may be greater than a thickness Th3 of the elastomer film F in the non-side edge portion Ac.

Preferably, at each of the bonded portions 8, some of a resin configuring the elastomer film F penetrates between plural fibers configuring the nonwoven fabric sheets S1, S2.

In this case, the thickness of the elastomer film F in the bonded portions 8 is thinner than the thickness of the elastomer film F in non-bonded portions 9 around the bonded portions 8.

Because the film is thin in this way, resistance to stretching in each of the bonded portions becomes smaller, and consequently it becomes easy to reduce variations in the elongation stress of the elastomer film.

For example, the laminate may configure at least part of a waist portion of a disposable wearable article, and the at least one side edge portion A_{E} may be an upper edge portion or a lower edge portion of the waist portion.

In this case, a situation where elongation stress becomes greater in the upper edge portion and/or the lower edge portion of the waist portion can be avoided and fit improves.

The features described and/or illustrated in association with one of the above aspects or the following embodiments can be used in the same or similar form in one or more other aspects or other embodiments and/or in combination with, or instead of, features of other aspects or embodiments.

### EMBODIMENTS

The present invention will become more clearly understood from the following description of preferred embodiments taken in conjunction with the accompanying drawings. However, the embodiments and the drawings are merely for the purposes of illustration and explanation and should not be utilized to define the scope of the present invention. The scope of the present invention shall be defined only by the claims. In the accompanying drawings, like parts numbers in multiple views refer to the same or corresponding parts.

It will be noted that some KATAKANA notations in the present specification are accompanied by English words in parentheses to make their meanings clearer.

An embodiment of the present invention will be described below in accordance with the drawings.

The stretchable laminate can be used for a variety of purposes. Below, a case where the laminate is applied to a disposable diaper (an example of a wearable article) will be exemplified and described. The diaper includes a waist portion that covers the waist of the wearer and a crotch portion that covers the crotch of the wearer. The laminate is used for the waist portion.

A detailed structure in a case where the stretchable laminate is used in a disposable diaper is, for example, disclosed in US 2013/0110073 A1 (WO 2012/017817 A1), and the entire disclosure thereof is incorporated herein.

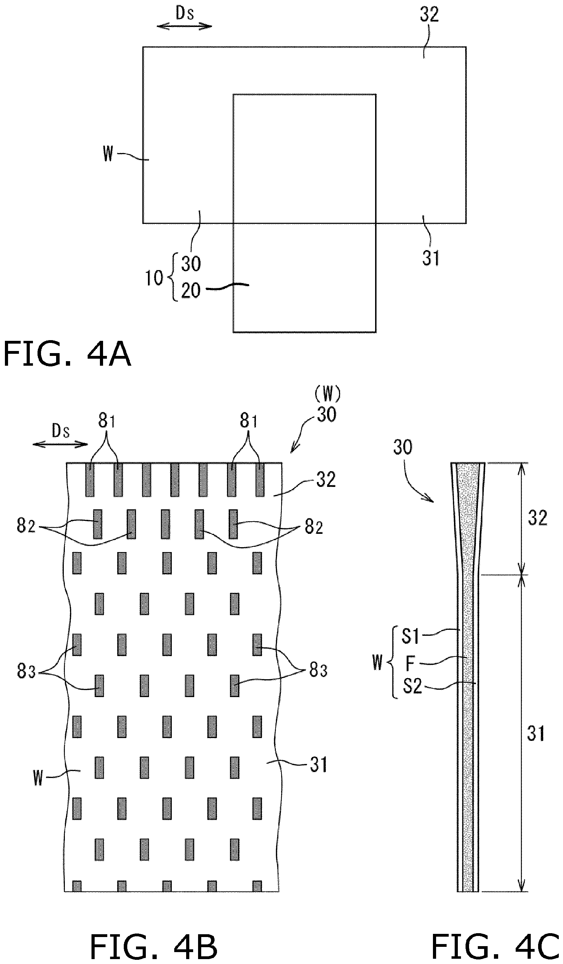

A laminate W shown in FIG. 4A to FIG. 4C is formed by sandwiching a thermoplastic stretchable elastomer film F between a pair of breathable nonwoven fabric sheets S1, S2 that include thermoplastic fibers.

The pair of nonwoven fabric sheets S1, S2 and the film F are laminated by being heat fused to each other at numerous bonded portions 8 (hereinafter called bonded portions 8 when, for example, collectively referring to bonded portions 8₁, 8₂, 8₃) shown in FIG. 4B. The laminate W can, for example, be used for the flaps and waist portion of the disposable diaper. In that case, the waist direction is the stretch direction Ds.

The bonded portions 8 may be formed by the vibrational energy of an ultrasonic horn or may be formed by being heated with a heating roll.

An example of the wearable article will be described before detailed description of the laminate.

FIG. 4A shows a wearable article 10.

As shown in this drawing, the wearable article 10 includes an absorbent body 20 and a waist portion 30. The absorbent body 20 is long in a longitudinal direction orthogonal to the waist direction. The wearable article may be the pants type of FIG. 4A or a diaper type.

The absorbent body 20 is provided with an absorbent core not shown in the drawings. The absorbent core absorbs bodily fluids. The absorbent core is sandwiched between a topsheet and a backsheet. The sheets and the absorbent core are laminated to each other.

The topsheet comprises a thin nonwoven fabric permeable to liquid and covers the skin side of the absorbent core. A cuff not shown in the drawings may be provided on the topsheet.

The backsheet covers the non-skin side of the absorbent core and comprises a resin sheet impermeable to liquid. The waist portion 30 is adhered to end portions in the longitudinal direction of the absorbent body 20.

The waist portion 30 is formed of the stretchable laminate W, extends in the waist direction of the wearer, and is configured to cover the waist of the wearer. The absorbent body 20 is configured to cover the crotch of the wearer.

FIG. 4B and FIG. 4C show a structural example of the laminate W configuring the waist portion 30.

Oblong first and second bonded portions 8₁, 8₂ are provided in a plurality of columns and a plurality of rows (two rows) in an upper edge portion 32 of the waist portion 30. Oblong third bonded portions 8₃ are provided in a plurality of columns and a plurality of rows (in a matrix) in a lower portion 31 of the waist portion 30. Each bonded portion 8 is intermittently arranged in the waist direction and intermittently arranged in the longitudinal direction.

Next, details about the structure of the laminate W will be described.

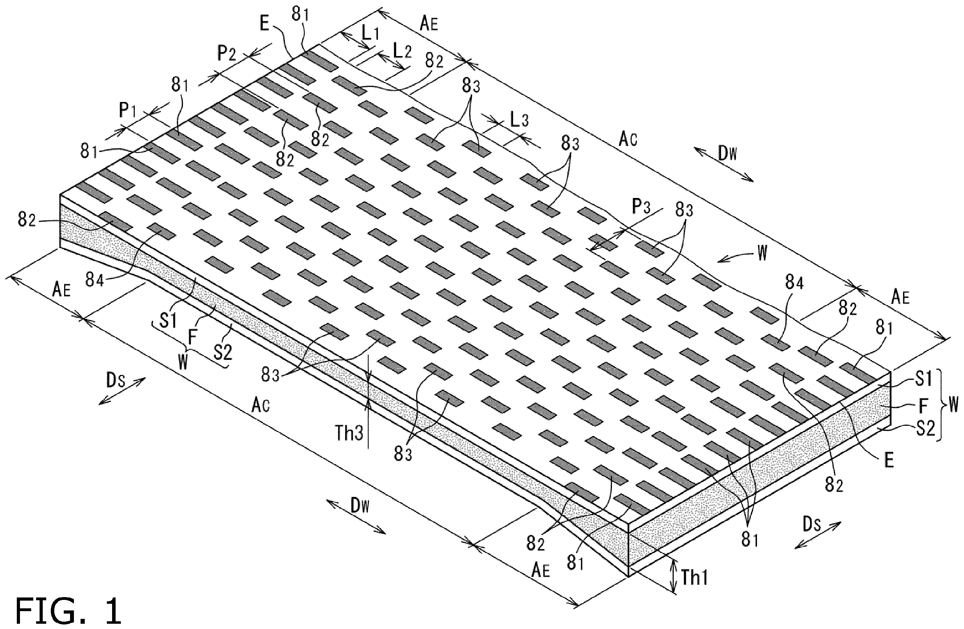

In the laminate W shown in FIG. 1, a non-side edge portion Ac is formed in a region (intermediate portion) between a pair of side edge portions A_{E}, A_{E}.

The laminate W shown in FIG. 1 is a laminate in which a thermoplastic elastomer film F having greater stretchability in a stretch direction Ds than in a width direction Dw orthogonal to the stretch direction Ds and first and second nonwoven fabric sheets S1, S2 are laminated to each other. In the case of the present example, the elastomer film F is sandwiched between the pair of sheets S1, S2, but the number of the nonwoven fabric sheets may be one.

A thickness Th1 of the elastomer film F in the side edge portions A_{E} on both sides is greater than a thickness Th3 of the elastomer film F in the non-side edge portion Ac.

In FIG. 1, the bonded portions 8, which are shown shaded in gray, are intermittently arranged in the width direction Dw and the stretch direction Ds, and the elastomer film F and the sheets S1, S2 are heat fused to each other at these bonded portions 8 and laminated to each other.

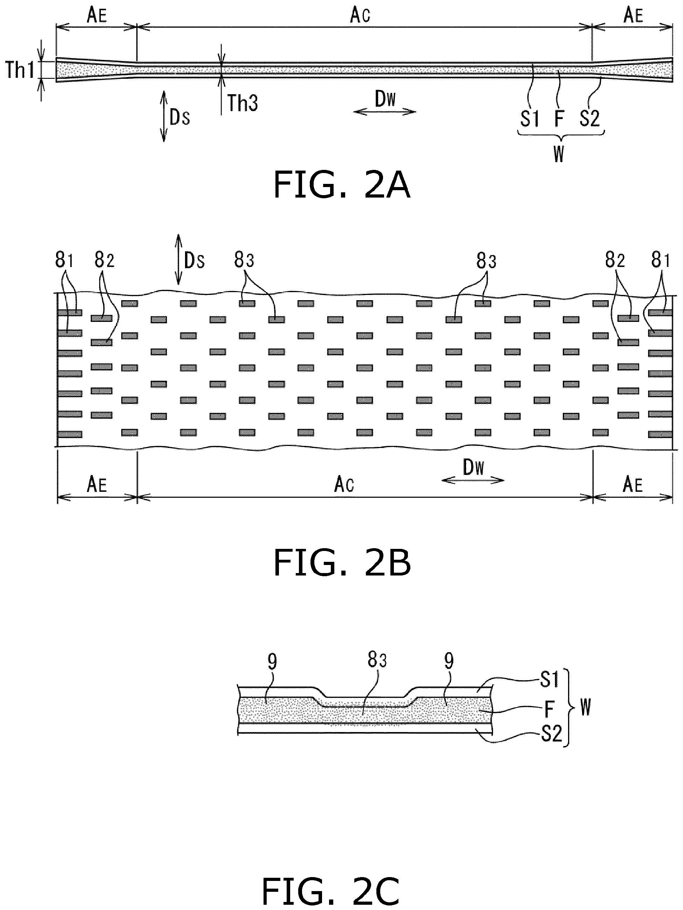

As indicated by halftone dots in FIG. 2C, at each of the bonded portions 8, some of a resin component configuring the elastomer film F penetrates between plural fibers configuring the nonwoven fabric sheets S1, S2. Consequently, the thickness of the elastomer film F in the bonded portions 8 is substantially thinner than the thickness of the elastomer film F in non-bonded portions 9 around the bonded portions 8.

The elastomer film F that is substantially thin in this way reduces the elongation stress of the laminate W.

As shown in FIG. 1, a length L (hereinafter called length L when, for example, collectively referring to lengths L₁, L₂, L₃) of each of the bonded portions 8 in the width direction Dw is longer than that of the bonded portions 8 in the stretch direction Ds. That is, each of the bonded portions 8 is for example a rectangle that is long in the width direction Dw. The bonded portions 8 having such a shape easily reduce variations in stress in the width direction Dw that occurs in the laminate W.

Here, a sum Σ1 of first lengths and a sum Σ2 of second lengths are set as follows.

Sum of first lengths Σ1: The sum of lengths of each of the bonded portions 8 in the width direction Dw in at least one side edge portion A_{E} in the width direction Dw per unit area of the laminate in that side edge portion A_{E}.

Sum of second lengths Σ2: The sum of lengths of each of the bonded portions 8 in the width direction Dw in the non-side edge portion Ac in the width direction Dw per unit area of the laminate W in that non-side edge portion Ac.

The sum Σ1 of the first lengths defined as described above is greater than the sum Σ2 of the second lengths. In order to set the sums of the lengths to such a relationship, in the present example the length L and arrangement pitches P of the bonded portions 8 are set as follows.

When for example collectively referring to the bonded portions, to the lengths, and to the arrangement pitches, for the sake of convenience subscript numbers are not added.

It will be noted that the bonded portions 8 may, for example, each be rhombic, elliptical, or hourglass-shaped. Furthermore, the shape of each of the bonded portions 8 may be a mixture of two or more shapes.

In FIG. 1, at least two rows of first bonded portions 8₁ and second bonded portions 8₂ intermittently arranged in the stretch direction Ds are provided in the side edge portions A_{E}. The first bonded portions 8₁ are provided in positions closest to edges E of the side edge portions A_{E}, and the row of the second bonded portions 8₂ is provided next to the row of the first bonded portions 8₁. Third bonded portions 8₃ are provided in a plurality of columns and a plurality of rows in the non-side edge portion Ac. It will be noted that fourth bonded portions 8₄ having the same shape and size as the third bonded portions 8₃ may be provided in parts of the regions of the side edge portions A_{E} as in the present example.

In order to obtain the aforementioned relationship between the sums of the lengths, in the present example the lengths and arrangement pitches of the first, second, and third bonded portions 8 are set as follows.

For example, the lengths of the bonded portions 8 in the width direction Dw are set as follows.

In FIG. 1, the lengths L₁, L₂ in the width direction Dw in the side edge portions A_{E} are greater than the length L₃ in the width direction Dw in the non-side edge portion Ac. More specifically, the length L₁ of the first bonded portions 8₁ in the width direction Dw is greater than the length L₂ of the second bonded portions 8₂ in the width direction Dw. Furthermore, the lengths L₁, L₂ of the first and second bonded portions 8₁, 8₂ in the width direction Dw are greater than the length L₃ of the third bonded portions 8₃ in the width direction Dw. It will be noted that the length L₂ of the second bonded portions 8₂ may be greater than the length L₁ of the first bonded portions 8₁.

The arrangement pitches of the bonded portions 8 in the width direction Dw are set as follows.

In FIG. 1, arrangement pitches P₁, P₂ of the bonded portions 8 in the stretch direction Ds in the side edge portions A_{E} are smaller than an arrangement pitch P₃ of the bonded portions 8 in the stretch direction Ds in the non-side edge portion Ac. More specifically, the arrangement pitch P₁ of the first bonded portions 8₁ in the stretch direction Ds is smaller than the arrangement pitch P₂ of the second bonded portions 8₂ in the stretch direction Ds. Furthermore, the arrangement pitches P₁, P₂ of the first and second bonded portions 8₁, 8₂ in the stretch direction Ds are smaller than the arrangement pitch P₃ of the third bonded portions 8₃ in the stretch direction Ds in one row in the non-side edge portion Ac. It will be noted that the arrangement pitch P₂ of the second bonded portions 8₂ may be smaller than the arrangement pitch P₁ of the first bonded portions 8₁.

It will be noted that it is desirable that the bonded portions 8 be arrayed in a staggered fashion so that they are not too close to each other.

It will be noted that the lengths L₁, L₂, L₃ of each of the first, second, and third bonded portions does not need to be the same. For example, the length L₁ of the first bonded portions may vary.

Furthermore, each of the arrangement pitches P₁, P₂, or P₃ may likewise also vary.

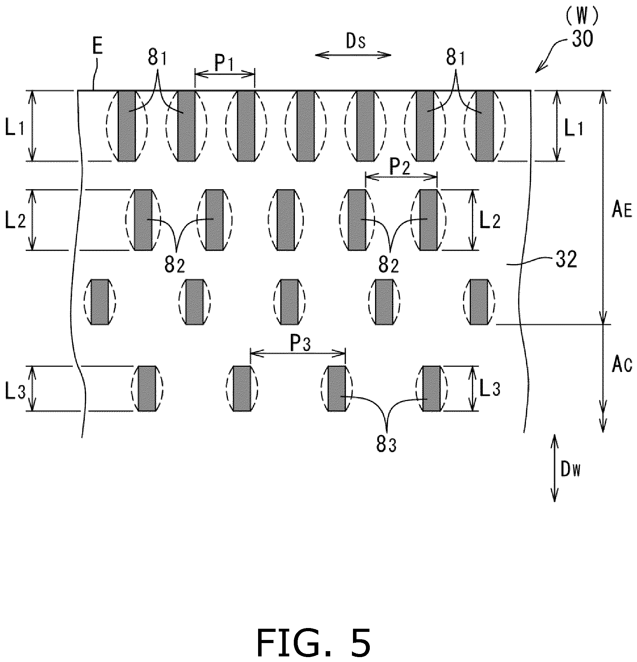

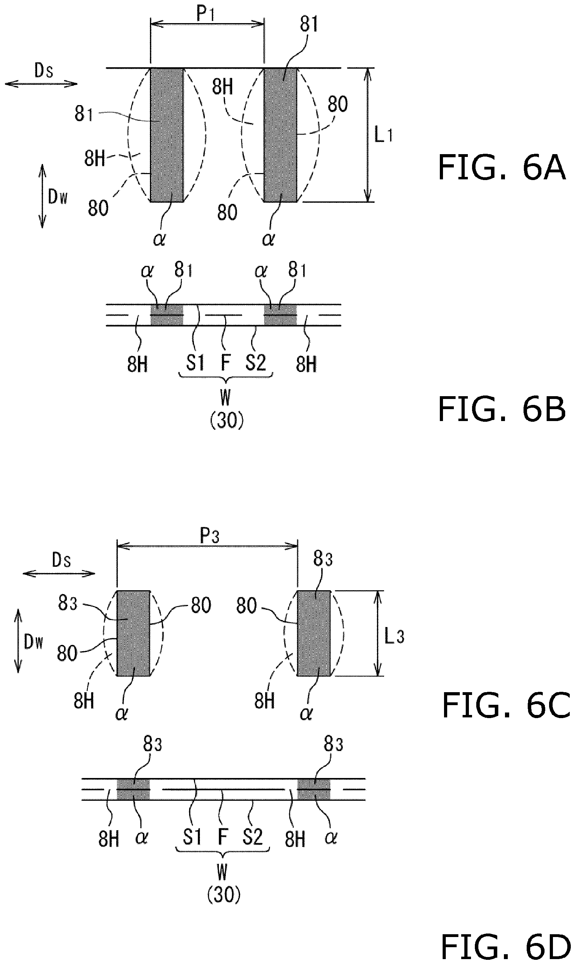

As shown in FIG. 5 and FIG. 6A to FIG. 6D, each bonded portion 8 of a plurality of bonded portions 8 at which the nonwoven fabric sheets S1, S2 and the elastomer film F are heat fused to each other includes a fused region α at which the sheets S1, S2 and the elastomer film F are heat fused to each other. The elastomer film F is in a broken state at boundary lines 80 defining the fused regions α.

The pair of nonwoven fabric sheets S1, S2 and the elastomer film F are heat fused to each other at the bonded portions 8 shaded in gray. The elastomer film F around the bonded portions 8 breaks during the heat fusion or breaks during re-elongation such as during wearing, so that holes 8H indicated by dashed lines appear in an elongated state of the elastomer film F in FIG. 5, FIG. 6B, and FIG. 6D. Consequently, as will be understood by comparing FIG. 6A and FIG. 6C, the longer the lengths L₁ to L₃ of the bonded portions 8 are, the larger the holes 8H are and the easier it is for the laminate to stretch.

Here, a first area ratio α1 and a second area ratio α2 are defined as follows.

First area ratio α1: Area ratio of the area of the bonded portions 8 in at least one side edge portion A_{E} in the width direction Dw to the unit area of the laminate in that side edge portion A_{E}.

Second area ratio α2: Area ratio of the area of the bonded portions 8 in the non-side edge portion Ac to the unit area of the laminate W in that non-side edge portion Ac.

The first area ratio α1 defined as described above is greater than the second area ratio α2.

Next, an example of a method of obtaining the waist portion from the laminate W and forming the wearable article will be briefly described.

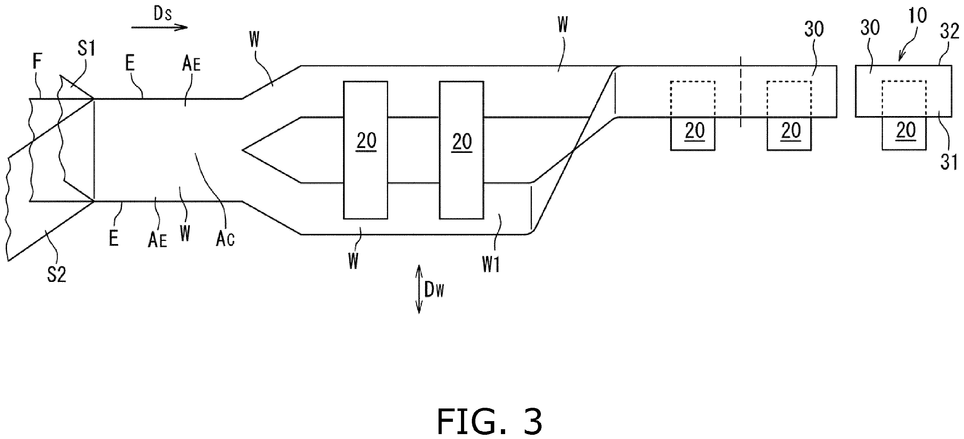

As shown in FIG. 3, the elastomer film F, which is extendable in a transport direction, is transported in the transport direction in a state in which it is stretched in the transport direction.

Nip rolls not shown in the drawings sandwich the elastomer film F having the pair of side edge portions A_{E} extending in the transport direction between the two sheets (continuous nonwoven fabric) S 1, S2, and bond the first sheet S1, the second sheet S2, and the elastomer film F so that they lie on top of each other to form the laminate W.

That is, the sheets S1, S2 and the elastomer film F are welded and bonded to each other at the numerous first to third bonded portions 8₁, 8₂, 8₃ disposed intermittently in the stretch direction Ds of FIG. 1 and intermittently in the width direction Dw. Here, the welding and bonding may be heat sealing (heat welding) or welding by ultrasonic energy.

As shown in FIG. 3, the laminate W having the pair of side edge portions A_{E} is slit by a slitter (not shown in the drawings) along the transport direction as it is being transported in the transport direction. Because of this, the laminate W becomes a pair of waist continuous bodies that become front and rear waist portions 30. That is, the laminate W having the non-side edge portion Ac between the pair of side edge portions A_{E} as in FIG. 1 is processed into the waist portion 30 in which the one side edge portion of FIG. 4B is the upper edge portion 32. After the slitting in FIG. 3, the two waist continuous bodies (laminates W) are relatively moved in the width direction Dw so as to separate from each other in the width direction Dw orthogonal to the transport direction.

Thereafter, as the pair of waist continuous bodies are transported in the transport direction generally parallel to each other, the absorbent body 20 is disposed (laid) so that the absorbent body 20 straddles the pair of waist continuous bodies and overlaps in part each waist continuous body, whereby a continuous laminate W1 is formed.

Thereafter, as shown in FIG. 3, the continuous laminate W1 is folded in two at the absorbent body 20 so that the pair of waist continuous bodies (laminates W) lie on top of each other.

Side seals are formed in end portions in the transport direction of the portions that become the front and rear waist portions 30, whereby the pair of waist continuous bodies are welded and bonded to each other. It will be noted that this welding and bonding may be welding by the ultrasonic energy of an ultrasonic horn or may be heat sealing.

Thereafter, the continuous laminate W1 is cut out into sizes (units) of individual wearable articles 10 along imaginary cutting lines indicated by the long dashed double-short dashed line. That is, in order to successively form the individual wearable articles 10, the continuous laminate W1 is successively cut between the absorbent main bodies 20 adjacent to each other in the transport direction. Because of this cutting, individual pants-type wearable articles10 shown in FIG. 4A are obtained.

In this way, the laminate configures at least part of the waist portion 30 of the disposable wearable article, and each side edge portion A_{E} becomes the upper edge portion 32 of the waist portion.

Next, an example of a method and apparatus for manufacturing the laminate W will be described.

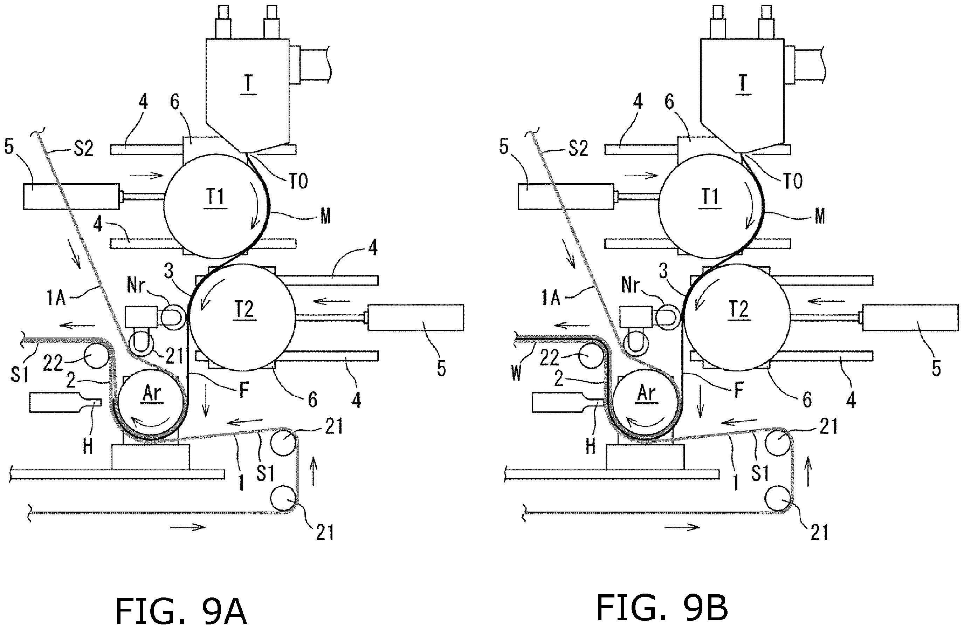

FIG. 9B shows a steady operation for continuously forming the laminate W. In this drawing, a discharge unit T is a known extruder called a T-die, and a thermoplastic elastomer (resin) in a molten state is temporarily stored in the T-die. The T-die discharges from a discharge outlet TO thereof into a film the resin in the molten state that becomes a pre-elastomer (an example of a film raw material) M to continuously form the pre-elastomer M.

The pre-elastomer M discharged from the discharge outlet TO becomes wrapped on the outer peripheral surface of a first cooling roll T1 and temporarily cooled, and is transported toward the outer peripheral surface of a second cooling roll T2 below the first cooling roll T1. Because of this, the pre-elastomer M generally solidifies and has elasticity (stretchability) as the elastomer film (an example of a thermoplastic film) F.

The pre-elastomer M headed toward the second cooling roll T2 is secondarily cooled by the outer peripheral surface of the second cooling roll T2. Because of this, the pre-elastomer M completely solidifies into the elastomer film (elastic film) F.

The secondarily cooled elastomer film F becomes sandwiched between the second cooling roll T2 and a nip roll Nr and then heads to a bonding roll Ar. The bonding roll Ar has a greater circumferential velocity (transport velocity) than the second cooling roll T2, and for that reason the elastomer film F is stretched in the transport direction between the nip roll Nr and the bonding roll Ar.

In this way, the molten resin becomes the elastomer film (thermoplastic film) F via the state of the pre-elastomer (film raw material) M. Here, the point in time at which the molten resin as a substance transitions to the pre-elastomer M and/or transitions from the pre-elastomer M to the elastomer film F varies depending on the glass transition temperature, the thickness of the resin, and the room temperature, and is not certain.

For example, although the molten resin turns into the pre-elastomer M just after exiting the discharge port TO and may appear at first glance to be solid, it may have properties close to those of a liquid without elasticity.

Furthermore, although the pre-elastomer M changes to the elastomer film F at the position where it is pulled downstream from the nip roll Nr, it may turn into the elastomer film F after coming into contact with the second cooling roll T2 upstream of the nip roll Nr or turn into the elastomer film F after coming into contact with the first cooling roll T1.

Film pass line 3 is a transport path for the film in a state in which the film is at least in part the pre-elastomer (film raw material) M.

Meanwhile, the first and second sheets S 1, S2 comprising nonwoven fabric are supplied to the bonding roll Ar. The sheets S 1, S2 are each supplied to the bonding roll Ar along a first pass line 1 or a second pass line 1A for the sheet.

The elastomer film F is introduced to the bonding roll Ar in a state in which it is sandwiched between the pair of sheets S1, S2. On the bonding roll Ar, the pair of sheets S1, S2 and the elastomer film F are bonded and laminated to each other by an ultrasonic horn H, whereby the laminate W is formed.

It will be noted that the laminate W may be formed by being heat welded with a heating roll rather than by ultrasonic bonding by the ultrasonic horn H.

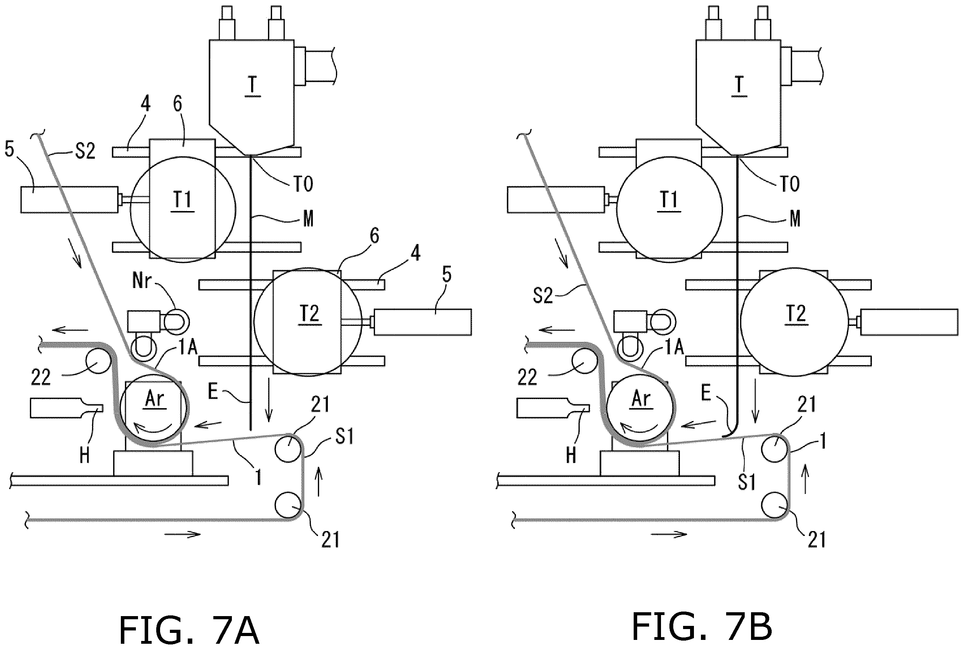

The production of the laminate W is continuously performed, but production may be temporarily stopped for size changes and the like. In this case, formation of the film pass line shown in FIG. 7A to FIG. 9A is executed in regard to the new elastomer film F.

Next, the manufacturing apparatus will be described.

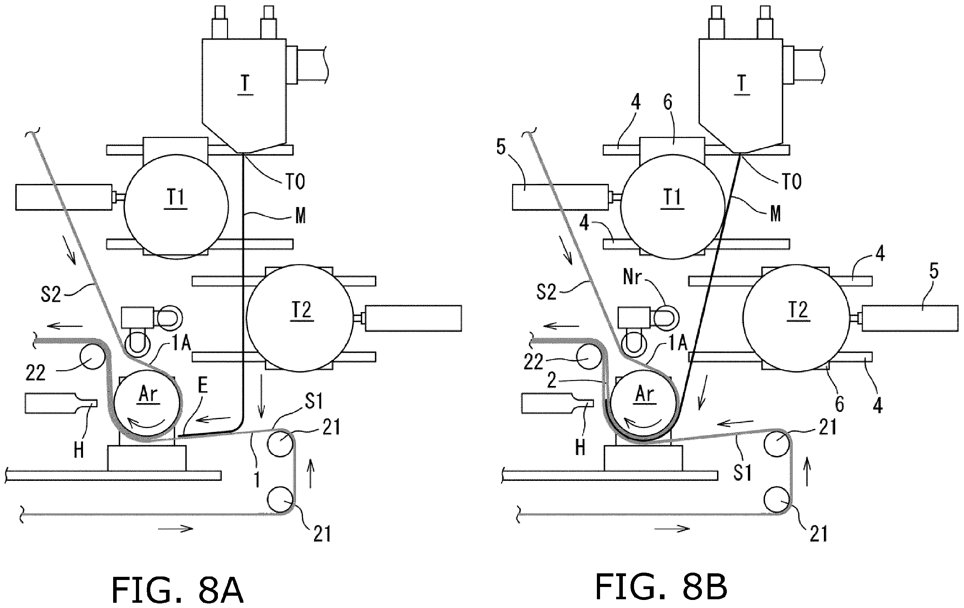

The cooling rolls T1, T2 of FIG. 8B are each rotatably supported on a slide base 6 and are moved in the horizontal direction as shown in FIG. 8B and FIG. 9A along a guider 4 by a cylinder 5. The cooling rolls T1, T2 are each driven to rotate at a circumferential velocity Vs by a motor not shown in the drawings. The bonding roll Ar is driven to rotate at a greater circumferential velocity V than the circumferential velocity Vs by a motor not shown in the drawings.

To newly form the film pass line 3 (FIG. 9B), first, the cooling rolls T1, T2 are retracted by the cylinders 5 as in FIG. 7A. Due to this retraction, the pre-elastomer M newly discharged and hanging down from the discharge outlet TO of the discharge unit T is in a state in which it is not in contact with the cooling rolls T1, T2. That is, the cooling rolls T1, T2 are provided so as to be capable of moving into contact with and away from the hanging pre-elastomer M of FIG. 7A.

After the process of forming the film pass line 3 of FIG. 9A, the ultrasonic horn H repeatedly ultrasonically vibrates relative to the bonding roll Ar as in FIG. 9B to thereby bond the elastomer film F to both sheets S1, S2 on the bonding roll Ar, whereby the laminate W is formed. That is, both sheets S1, S2 and the elastomer film F are transported on top of each other along the outer peripheral surface of the bonding roll Ar, and the ultrasonic horn H ultrasonically vibrates relative to the bonding roll Ar so that ultrasonic energy is applied to both sheets S1, S2 and the elastomer film F, whereby the nonwoven fabric sheets and the elastomer film are bonded and laminated.

This bonding is executed as a result of the elastomer film F, whose temperature has increased by being heated by ultrasonic energy, heat fusing to the nonwoven fabrics sheets. At the same time, the bonding is performed in a state in which tensile force acts on the film and the sheets due to the difference in velocity between the circumferential velocity Vs of the second cooling roll T2 and the circumferential velocity V of the bonding roll Ar. For that reason, the elastomer film F that has softened due to its temperature increasing is stretched in the stretch direction Ds in FIG. 5 and FIG. 6A to FIG. 6D and breaks at the boundary lines 80 extending along the width direction Dw as indicated by dashed lines, whereby the holes 8H are formed in the elastomer film F of the laminate W.

While preferred embodiments have been described above with reference to the drawings, a variety of obvious changes and modifications will readily occur to those skilled in the art upon reading the present specification.

For example, just the lower edge portion of the waist portion may have the structure of the side edge portion. Furthermore, the upper and lower edge portions of the waist portion may have the structure of the side edge portion without slitting the laminate in two. Furthermore, the bonded portions may be circular or square for example.

Consequently, such changes and modifications are to be interpreted as being within the scope of the present invention as defined by the claims.

### INDUSTRIAL APPLICABILITY

The present invention can be applied to the manufacture of a laminate for wearable articles such as disposable diapers.

### REFERENCE SIGNS LIST

1: First Pass Line for Sheet
1A: Second Pass Line for Sheet
2: Laminate Pass Line
3: Film Pass Line
4: Guiders
5: Cylinders
6: Slide Bases
8, 8₁, 8₂, 8₃: Bonded Portions
8H: Holes
80: Boundary Lines
10: Wearable Article
20: Absorbent Body
30: Waist Portion
31: Lower Portion
32: Upper Edge Portion
21: First Rolls
22: Second Roll
Ac: Non-side Edge Portion
A_{E}: Side Edge Portions
Ds: Stretch Direction
Dw: Width Direction
F: Elastomer Film (Example of Thermoplastic Film)
M: Pre-elastomer (Example of Film Raw Material)
S1: First Sheet
S2: Second Sheet
W: Laminate
E: Edges
Ar: Bonding Roll
H: Ultrasonic Horn
Nr: Nip Roll
L, L₁, L₂, L₃: Lengths
P, P₁, P₂, P₃: Pitches
T: Discharge Unit
TO: Discharge Outlet
T1: First Cooling Roll
T2: Second Cooling Roll
Th1, Th3: Thicknesses
α: Fused Regions
α1, α2: Area Ratios

## Claims

1. A laminate W comprising a thermoplastic elastomer film F having greater stretchability in a stretch direction Ds than in a width direction Dw orthogonal to the stretch direction Ds and nonwoven fabric sheet S1, S2 that are laminated to each other,
wherein the elastomer film F and the sheet S1, S2 are laminated by being heat fused to each other at bonded portions 8 intermittently arranged in the width direction Dw and the stretch direction Ds, and
the sum of lengths of each of the bonded portions 8 in the width direction Dw in at least one side edge portion A_{E} in the width direction Dw per unit area of the laminate in that side edge portion A_{E} is greater than the sum of lengths of each of the bonded portions 8 in the width direction Dw in a non-side edge portion Ac in the width direction Dw per unit area of the laminate W in that non-side edge portion Ac.

2. The laminate according to claim 1, wherein a length L of each of the bonded portions 8 in the width direction Dw is longer than that of the bonded portions 8 in the stretch direction Ds.

3. The laminate according to claim 2, wherein length L₁, L₂ in the width direction Dw in the side edge portion A_{E} are longer than a length L₃ in the width direction Dw in the non-side edge portion Ac.

4. The laminate according to claim 3, wherein
at least two rows of first bonded portion 8₁ and second bonded portion 8₂ intermittently arranged in the stretch direction Ds are provided in the side edge portion A_{E},
the first bonded portions 8₁ are provided in positions closest to an edge E of the side edge portion A_{E}, and
the row of the second bonded portions 8₂ is provided next to the row of the first bonded portions 8₁.

5. The laminate according to claim 4, wherein a length L₁ of the first bonded portions 8₁ in the width direction Dw is longer than a length L₂ of the second bonded portions 8₂ in the width direction Dw.

6. The laminate according to claim 5, wherein
a plurality of third bonded portions 8₃ are provided in the non-side edge portion Ac, and
the length L₂ of the second bonded portions 8₂ in the width direction Dw is longer than a length L₃ of the third bonded portions 8₃ in the width direction Dw.

7. The laminate according to any one of claims 2 to 6, wherein an arrangement pitch P₁, P₂ of the bonded portions 8 in the stretch direction Ds in the side edge portion A_{E} is smaller than an arrangement pitch P₃ of the bonded portions 8 in the stretch direction Ds in the non-side edge portion Ac.

8. The laminate according to claim 4, wherein an arrangement pitch P₁ of the first bonded portions 8₁ in the stretch direction Ds is smaller than an arrangement pitch P₂ of the second bonded portions 8₂ in the stretch direction Ds.

9. The laminate according to claim 6, wherein an arrangement pitch P₂ of the second bonded portions 8₂ in the stretch direction Ds is smaller than an arrangement pitch P₃ of the third bonded portions 8₃ in the stretch direction Ds in one row in the non-side edge portion Ac.

10. The laminate according to any one of claims 1 to 9, wherein a first area ratio α1 of the area of the bonded portions 8 in at least one side edge portion A_{E} in the width direction Dw to the unit area of the laminate in that side edge portion A_{E} is greater than a second area ratio α2 of the area of the bonded portions 8 in a non-side edge portion Ac in the width direction Dw to the unit area of the laminate W in that non-side edge portion Ac.

11. The laminate according to any one of claims 1 to 10, wherein
each bonded portion 8 of a plurality of bonded portions 8 at which the nonwoven fabric sheet S1, S2 and the elastomer film F are heat fused to each other includes a fused region α at which the sheet S1, S2 and the elastomer film F are heat fused to each other, and
the elastomer film F is in a broken state at boundary lines 80 defining the fused regions α.

12. The laminate according to any one of claims 1 to 11, wherein a thickness Th1 of the elastomer film F in the at least one side edge portion A_{E} is greater than a thickness Th3 of the elastomer film F in the non-side edge portion Ac.

13. The laminate according to any one of claims 1 to 12, wherein at each of the bonded portions 8, some of a resin configuring the elastomer film F penetrates between plural fibers configuring the nonwoven fabric sheet S1, S2.

14. The laminate according to claim 13, wherein the thickness of the elastomer film F in the bonded portions 8 is thinner than the thickness of the elastomer film F in non-bonded portions 9 around the bonded portions 8.

15. The laminate according to any one of claims 1 to 14, wherein
the laminate configures at least part of a waist portion 30 of a disposable wearable article 10, and
the at least one side edge portion A_{E} is an upper edge portion 32 or a lower edge portion of the waist portion 30.
